# EUROPEAN PATENT APPLICATION

(11) **EP 3 714 883 A1**
(43) Date of publication of application: **30.09.2020**
(21) Application number: 19164877.3
(22) Date of filing: 25.03.2019
(51) Int. Cl.: A61K 31/403, A61K 31/437, A61P 35/00

(54) **INDOLE CONTAINING SMALL CHEMICAL COMPOUNDS AND USE THEREOF FOR THE NON-CYTOTOXIC AND IMMUNOLOGICAL TREATMENT OF CANCER**

(71) Applicant: AC BioScience SA, 1024 Ecublens (CH)
(72) Inventor: AUCLAIR, Christian, 78730 Saint Arnoult en Yvelines (FR)
(74) Representative: KATZAROV S.A.

(57) **Abstract**

The invention provides an indole-containing compound able to induce actin network remodeling in cancer cells into actin organized network and use thereof in non-cytotoxic cancer immunotherapy, for enhancing immune checkpoint inhibitor therapy, for activating cancer immune response, for treating or lessening the symptoms of, or preventing the human cancer, alone or in combination with one or more cancer immunotherapeutic agent for simultaneous, separate or sequential administration.

## Description

### FIELD OF THE INVENTION

The invention provides an indole-containing compound able to induce actin network remodeling in cancer cell into actin organized network and use thereof in non-cytotoxic cancer immunotherapy, for enhancing immune checkpoint inhibitor therapy, for activating cancer immune response, for treating or lessening the symptoms of, or preventing the human cancer, alone or in combination with one or more cancer immunotherapeutic agent for simultaneous, separate or sequential administration.

### BACKGROUND OF THE INVENTION

Large amount of data has been accumulated on how normal cells become malignant. The deregulation of the cell machinery leading to malignant transformation is known to involve oncogenes, tumor suppressor genes, DNA repair machinery, microenvironment, tumor neoangiogenesis, metabolism, chromosomal instability and immune silencing. Mainly due to their genetic instability, it has been observed that tumor cells may spontaneously lose their malignant phenotype leading to the so-called tumor reversion. Spontaneous tumor reversion occurs at a very low rate, but genetic or pharmacological manipulations have demonstrated the feasibility of tumor reversion control. As example, tumor reversion can be experimentally achieved by the downregulation of oncogenic drivers, by inhibition of integrin β1 signaling or by the inhibition of TCTP.

Actin microfilament is ubiquitous protein polymer present in all eukaryotic cells. As one of the major proteins in the cell, actin and its associate proteins play important structural an functional role, such as maintaining cell morphology, cell adhesion, motility, exo- and endocytosis, as well as cell division. Growing evidences show that alteration of actin polymerization, or actin remodelling could play a pivotal role in the regulation of morphologic and phenotypic changes leading to malignancies. Tumor cells frequently present a disorganized actin homeostasis resulting in the activation of numerous signalling pathways involving oncogene products such as Src, Abl or most notably small GTPases of Ras superfamily proteins (Rac, Rho and Cdc42) involved in actin remodelling. In cancer cells, disruption of the actin cytoskeleton network results in the impaired functionality of membrane adhesion proteins such as cadherins, integrins, ICAM-1 etc...which in turn decreases the cell-cell adhesion forces and the cell-extracellular matrix adhesion as well as the stability of immunological synapses either between MHC-1 expressing cells and CD8 lymphocytes or APC and ThCD4 lymphocytes. Importantly, the MHC-1 restricted antigen presentation pathway is down-regulated in many different cancer tissues and cancer cell lines. This has led to the hypothesis that the defective pathway may have a significant role in loss of immuno-surveillance and possibly in causation of cancer progression.

Therefore, there is still a need for a treatment that can efficiently treat cancer.

### SUMMARY OF THE INVENTION

An aspect of the present invention provides a pharmaceutical combination of an indole-containing compound able to induce actin network remodelling in cancer cells into actin organized network and one or more cancer immunotherapeutic agent for simultaneous, separate or sequential administration,
wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts,
and wherein the cancer immunotherapeutic agent is selected from the group comprising immune checkpoint inhibitor, TCR-T cells, CAR-T cells or combinations thereof; preferably the cancer immunotherapeutic agent is an immune checkpoint inhibitor.

Another aspect of the present invention provides a pharmaceutical combination of the invention for use in a method of activating cancer immune response.

Another aspect of the present invention provides a pharmaceutical combination of the invention for use in non-cytotoxic method of treating or lessening the symptoms of human cancer, or preventing human cancer progression.

Another aspect of the present invention provides an indole-containing compound able to induce actin network remodelling in cancer cells into actin organized network for use in a non-cytotoxic cancer immunotherapy method, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

Another aspect of the present invention provides an indole-containing compound able to induce actin network remodelling in cancer cells into actin organized network for use in a method of activating cancer immune response, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

Another aspect of the present invention provides an indole-containing compound able to induce actin network remodelling in cancer cells into actin organized network for use in a non-cytotoxic method of treating or lessening the symptoms of human cancer, or preventing human cancer progression, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

### BRIEF DESCRIPTION OF THE FIGURES

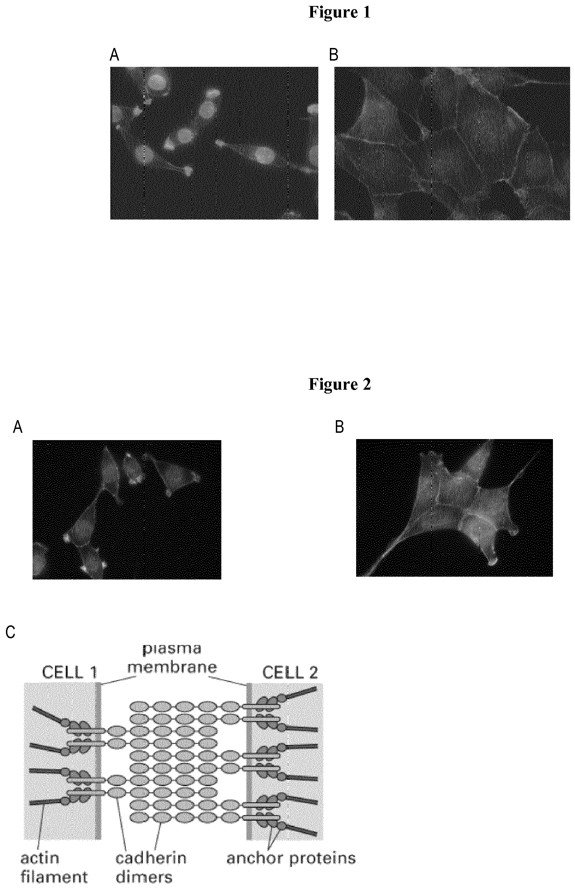

**Figure 1** shows A: Murine malignant fibroblasts expressing the fusion gene EWS-Fli1. B: fibroblasts as in A following the treatment with the ACB-1E methanolic extract. Red color: actin.
**Figure 2** shows A: Murine malignant fibroblasts expressing the fusion gene EWS-Fli1. B: fibroblasts as in A following the treatment with the ACB-1E methanolic extract. Green color: actin, yellow color: β-catenin. C: Schematic representation of cell-cell adhesion; green color: cadherin, blue color β-catenin, red color: actin filament.

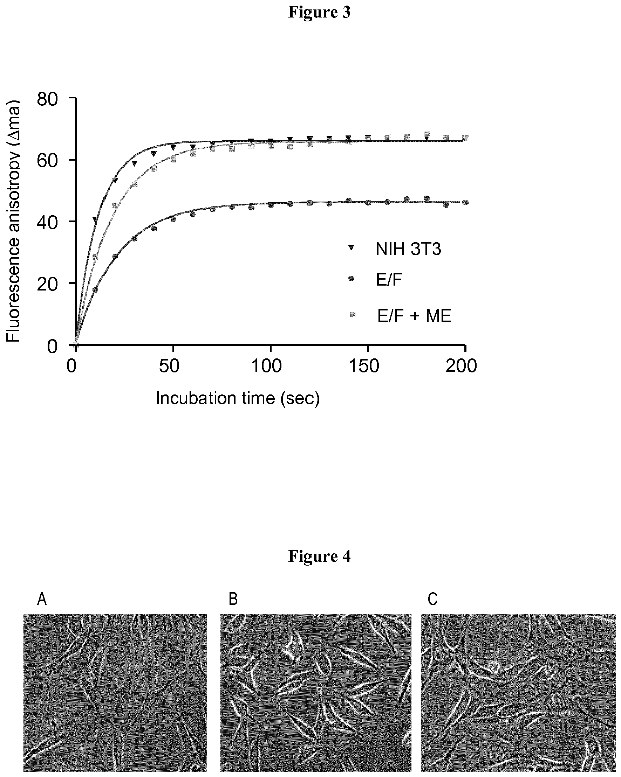

**Figure 3** shows effect of methanolic extract (ME) from ACB-1E on actin polymerization in malignant fibroblasts (E/F) extract. Polymerisation buffer and cellular extract were added at time zero and anisotropy enhancement was followed at 22°C by Alexa 488 actin. Measurements were made every 10 seconds. Final protein concentration in reaction mixtures was adjusted at 0.2 mg/ml. The curve was fitted according to the equation Y = Ymax.[1- exp (-K.x)]. Data represent mean ± standard deviation; n= 4. (▼) Cell extract from NIH-3T3, (●) Cell extract from EF, (■) Cell extract from EF plus 5mg/L methanolic extract from ACB-1E. E/F is referred to NIH-3T3 cells expressing the oncogenic protein EWS-Fli1.
**Figure 4** shows cell-cell adhesion rescue in malignant fibroblasts treated by ACB1801. A: non-malignant NIH 3T3 fibroblasts. B: Malignant NIH 3T3 fibroblasts expressing the fusion gene EWS-Fli1. C: malignant NIH 3T3 fibroblasts treated by 10µM ACB1801 for 48 hours.

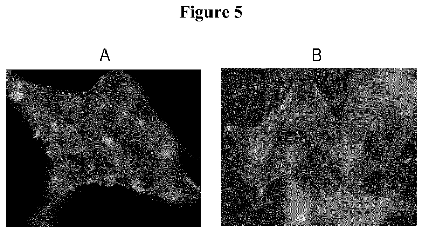

**Figure 5** shows A: Murine melanoma B16 F10 cells. B: Melanoma B16 F10 cells after treatment with 5µM ACB1801 for 24 hours.

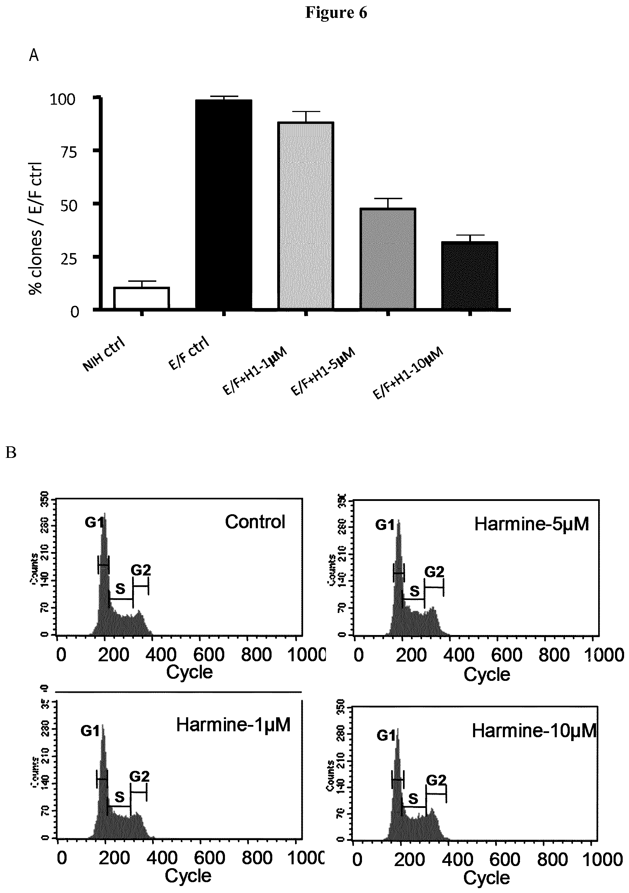

**Figure 6** shows A) Effect of ACB1801 on tumour cell growth in semi-solid medium (anchorage independent proliferation). Malignant fibroblasts (E/F) cells were seeded per 35-mm dishes in culture medium supplemented with methyl cellulose (E/F referred to malignant fibroblasts expressing EWS-Fli-1 oncogenic protein) . Clones with a diameter larger than 120µm were counted after 4 weeks. Values +/- SD were the results of three different experiments. B) Flow cytometry analysis of E/F cells cultured for 72 hours using standard operating conditions in the presence of increasing concentrations of ACB1801. Cells were labelled with propidium iodide and the % of cells in either G1/S/G2 phases was estimated.

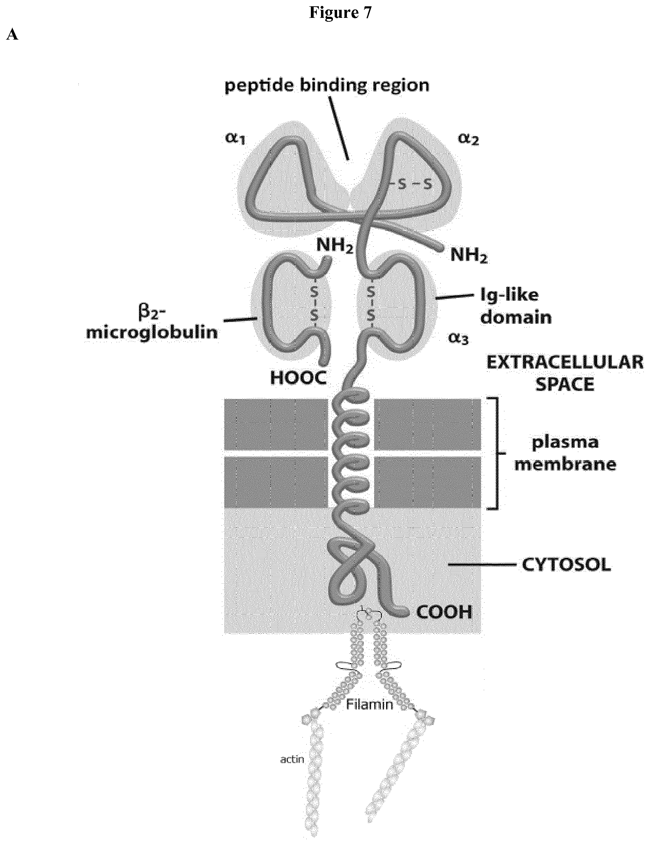

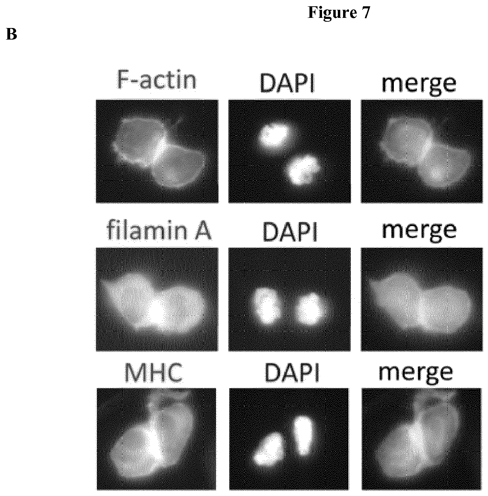

**Figure 7** shows A: Structure of MHC Class I. The two globular domains furthest from the plasma membrane that form the peptide binding region (PBR) are shaded in blue. The two Ig-like domains are shaded in grey. Figure adapted from Alberts et al. 2008: Figure 25-50 Molecular Biology of the Cell 5/e (©Garland Science 2008). The proposed binding of filamin (pink) actin as bridge between intrasellar domain of MHC I and F-actin has been added to the original figure. B: colocalization of MHC and filamin.

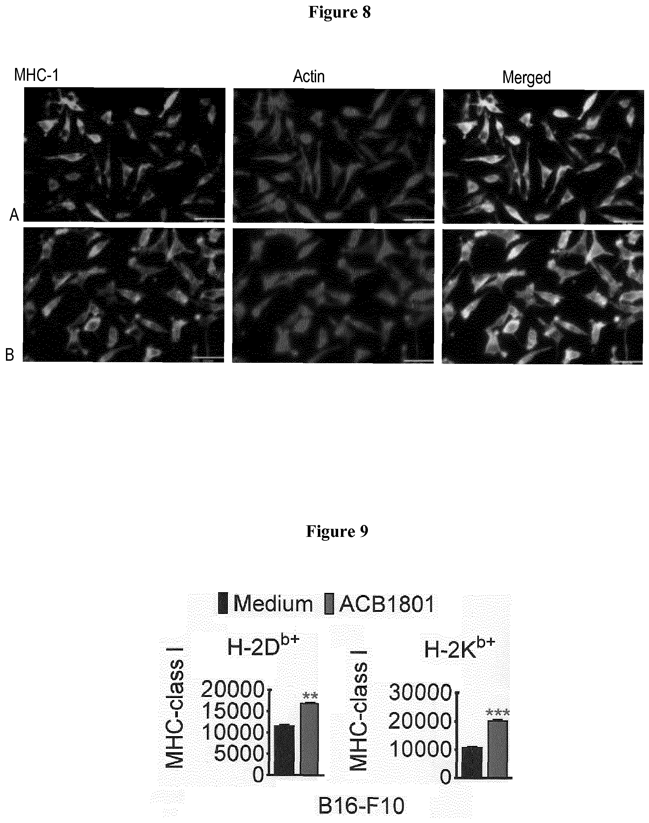

**Figure 8** shows immunocytochemistry for L929 MHC class I Stained (Green) and actin stained(Red) images: L 929 control (a), L929+ IFN- γ (b). IFN-γ pre-treated cells showed more fluorescent intensity when compared to cells only. Bar markers indicate 50 µm. (from Prasanthi KumchalaM.Sc., Acharya Nagarjuna University, 2006)
**Figure 9** shows effect of ACB1801 (Harmine) on the HMC I alloantigens (haplotype b) expression level at the melanoma B16 cell membrane. Melanoma B16 cells were treated by 5µM harmine for 24 hours. HMC I level was assessed by flow cytometry using the FMI mode.

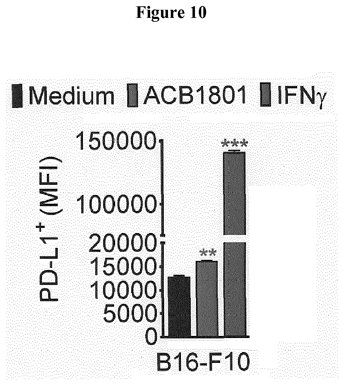

**Figure 10** shows comparative effect of harmine and interferon gamma on the PD-L1 expression level. Melanoma B16 cells were treated by 5µM harmine or 50µg/ml interferon for 24 hours. PD-L1 level was assessed by flow cytometry using the FMI mode.

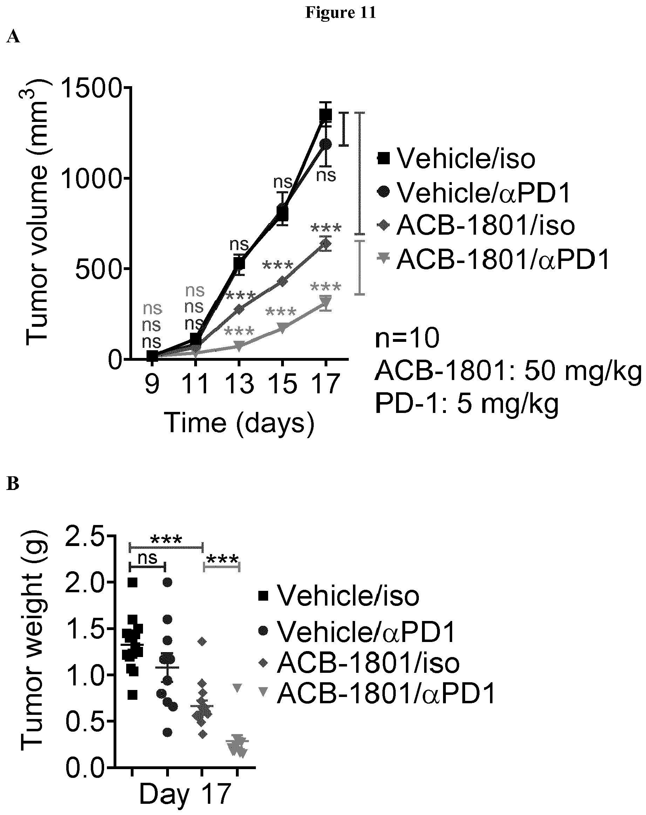

**Figure 11** shows tumour growth (A panel) and weight in g (B panel) of B16-F10 melanoma in control mice (vehicle/iso), and in mice treated with either anti-PD-1 alone (vehicle/αPD-1), ACB-1801 alone (ACB-1801/iso), or a combination of ACB-1801 and anti-PD-Ll (ACB-1801/αPD-1). Tumour volume was measured at the indicated time points. Tumour weight was determined on day 17. Results are reported as the average of 10 mice per group from 2 independent experiments conducted with 5 mice per group. Data are shown as mean ± SEM (error bars). Statistically significant differences (indicated by asterisks) are calculated using an unpaired two-tailed Student's t-test (ns = not significant, *** = P < 0.0005).

### DETAILED DESCRIPTION OF THE INVENTION

All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. Also as used in the specification and claims, the language "comprising" can include analogous embodiments described in terms of "consisting of" and/or "consisting essentially of".

As used in the specification and claims, the singular form "a", "an" and "the" include plural references unless the context clearly dictates otherwise.

As used in the specification and claims, the term "and/or" used in a phrase such as "A and/or B" herein is intended to include "A and B", "A or B", "A", and "B".

As used herein the terms "subject" and "patient" are well-recognized in the art, and, are used herein to refer to a mammal, and most preferably a human. In some embodiments, the subject is a subject in need of treatment or a subject having cancer or an immune disorder, who is likely to benefit from a treatment with combination therapy of the present invention. The term does not denote a particular age or sex. Thus, adult and newborn subjects, whether male or female, are intended to be covered.

As used herein the term "pharmaceutically acceptable excipients and/or carriers" means that the compositions or components thereof so described are suitable for use in contact with a mammal body, preferably human body, or suitable for any other means of administration to human body without undue toxicity, incompatibility, instability, irritability, allergic response, and the like. The term includes any and all solvents, dispersion media, coatings, antibacterial and antifungal agents, isotonic and absorption delaying agents and the like. The use of such media and agents for pharmaceutically active substances is well known in the art. In addition, various adjuvants such as are commonly used in the art may be included. Considerations for the inclusion of various components in pharmaceutical compositions are described, for example, in Gilman et al. (Eds.) (1990); Goodman and Gilman's: The Pharmacological Basis of Therapeutics, 8th Ed., Pergamon Press, which is incorporated herein by reference in its entirety.

The term "treat" and its grammatical variants (for example "to treat," "treating," and "treatment") refer to administration of an active pharmaceutical ingredient or the combination therapy of a present invention to a subject with the purpose of ameliorating or reducing the incidence of one or more symptoms of a condition or disease state in the subject. Such symptoms may be chronic or acute; and such amelioration may be partial or complete. In the present context, treatment entails administering the pharmaceutical combination of the invention to a subject.

The term "therapeutically effective amount," as used herein, refers to any amount of a specific component or combination of components that will cause a reduction of symptoms, disappearance of the symptoms or relief from symptoms related to for example cancer, when applied, either once, or repeatedly over time. For example, a therapeutically effective amount of a cancer immunotherapeutic agent, such as an immune checkpoint inhibitor, is an amount sufficient to effect beneficial or desired clinical results and/or sufficient to ameliorate, stabilize, reverse, slow and/or delay progression of cancer or immune disorder or to cure cancer or immune disorder. For example, a therapeutically effective amount of an indole-containing compound able to induce actin network remodeling in cancer cells into actin organized network is a non-cytotoxic amount sufficient to induce actin network remodeling in cancer cells into actin organized network in cells. Therapeutically effective amounts, which are non-cytotoxic amounts, can be readily determined by persons skilled in the art using routine experimentation and using tests and measures commonly employed in the art, or can be based upon the subjective response of patients undergoing treatment.

The term "tumour" and the term "cancer" are used interchangeably and both refer to an abnormal growth of tissue that results from excessive cell division.

The tumour phenotype maintenance is governed by the alteration of tumour cell communication with their environment including cell-cell communication, cell-extracellular matrix communication and cell-CTL communication. Restoring those communications may result in tumour phenotype reversion and may reactivate immune response. The efficient communications between tumour cells and their environment is mainly controlled by the membrane expression and functionality of adhesion molecules which in turn are under the dependence of the integrity of actin cytoskeleton network. The changes in the cytoskeletal architecture which is one of the main molecular mechanisms underlying malignant transformation could be a pertinent target process. Consequently, pharmacological-induced actin network rearrangement in tumour cells may result in the loss of malignant characters thanks to the rescue of adhesion and motility controls.

Indeed, most tumour cells display a dramatic alteration of actin dynamics resulting in a decrease of actin under F form. This cytoskeleton remodelling results in the decrease of the membrane residence time of adhesion proteins and in their abnormal orientational order subsequently leading to disappearance of contact inhibition, to change in integrin signalling and in the alleviation of immunological synapse efficiency. It should be emphasized that cell cytoskeletal remodelling is a key regulatory component of normal immunological synapse formation, likely through consolidation of adhesive interaction and modulation of architectural synapse stability. It is therefore proposed that, in cancer cells, the pharmacological remodelling of the actin network will rescue all adhesion-dependent signalling processes leading so to the malignant phenotype reversion (i.e. tumour reversion).

Indeed, compared to normal cells, malignant cells are characterized by an impairment of cytoskeleton architecture mainly due to abnormal actin dynamics regulation. The expression of players in most functions implicated in actin dynamics are affected: β-thymosins (monomer sequestering), p41Arp2/3, cortactin, WASP, profilin (filament nucleation and elongation), gelsolin, cofilin (filaments capping, severing or depolymerization). This impaired cytoskeleton organization is accompanied by an increased cell motility, a decrease of cell-cell adhesion forces, a loss of contact growth inhibition, a modification of intracellular trafficking and a decreased expression at the membrane level of various proteins including adhesion proteins, integrins and MHC-1 complex. All these features are the hallmarks of tumour phenotype responsible for tumour cell proliferation and invasion as well as for their inability to efficiently activate the cytotoxic T lymphocytes.

The present invention discloses indole-containing compounds, specifically indole-containing compounds of β-carboline family and carbazole family, able at non-cytotoxic concentrations, to modify the actin dynamics parameters leading to the decrease of cell motility, to recovery of cell-cell adhesion forces, to increase of the expression of MHC-1 complex at the cell membrane and globally to revert the tumour phenotype. The present invention discloses the effect of indole-containing compounds, such as harmine, on the cancer cells cytoskeleton remodelling, which in turn revert the tumour phenotype with a subsequent recovery of cancer cells immune recognition.

The present invention relates to the use of the compounds of the invention by themselves as antitumor agents, or in combination with non-cytotoxic cancer immunotherapy, such as immune checkpoint inhibitor therapy, TCR-T cells therapy, CAR-T cells therapy or combinations thereof to improve non-cytotoxic cancer immunotherapy, particularly in patients resistant to cancer immunotherapy, or in poor responder patients to cancer immunotherapy or in relapsed patients after cancer immunotherapy. Preferably, the cancer immunotherapeutic agent is an immune checkpoint inhibitor.

An aspect of the present invention provides the combined use of (i) an indole-containing compound able to induce actin network remodelling in cancer cells into actin organized network, and (ii) one or more cancer immunotherapeutic agents in methods of the present invention, wherein the indole-containing compound is administered to the subject before, during or after administration of the one or more cancer immunotherapeutic agent.

Thus, the invention provides a pharmaceutical combination of an indole-containing compound able to induce actin network remodelling in cancer cells into actin organized network and one or more cancer immunotherapeutic agent for simultaneous, separate or sequential administration,
wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts,
and wherein the cancer immunotherapeutic agent is selected from the group comprising immune checkpoint inhibitor, TCR-T cells, CAR-T cells or combinations thereof; preferably the cancer immunotherapeutic agent is an immune checkpoint inhibitor.

The invention further provides a kit comprising combination of an indole-containing compound able to induce actin network remodelling in cancer cells into actin organized network, one or more cancer immunotherapeutic agent, and an information leaflet containing written instructions for administering the indole-containing compound and the one or more cancer immunotherapeutic agent,
wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts,
and wherein the cancer immunotherapeutic agent is selected from the group comprising immune checkpoint inhibitor, TCR-T cells, CAR-T cells or combinations thereof; preferably the cancer immunotherapeutic agent is an immune checkpoint inhibitor.

It will be appreciated that the individual compounds of the pharmaceutical combination may be administered simultaneously, either in the same formulation or different pharmaceutical formulations, separately or sequentially. If there is separate or sequential administration, the delay in administering the one or more cancer immunotherapeutic agent should not be such as to lose the benefit of any synergistic therapeutic effect of the combination of the indole-containing compounds and the one or more cancer immunotherapeutic agent.

In an embodiment of the pharmaceutical combination of the present invention, the indole-containing compound is selected from the group comprising

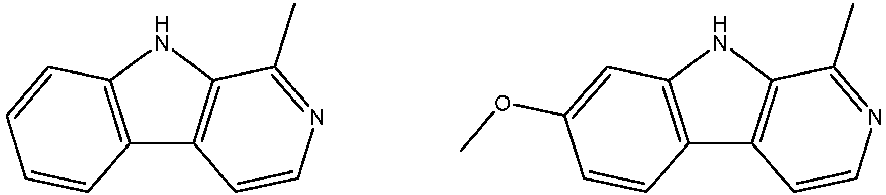

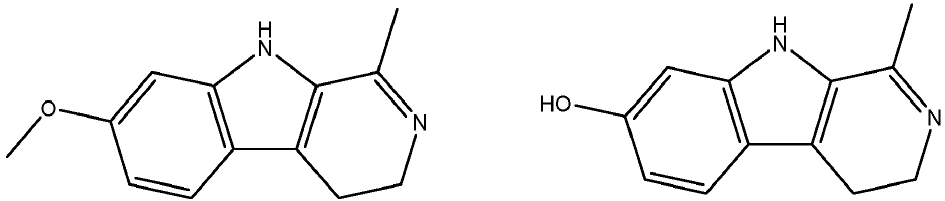

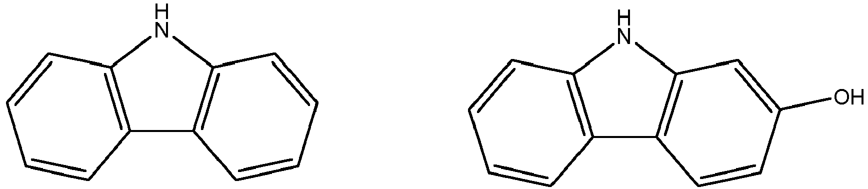

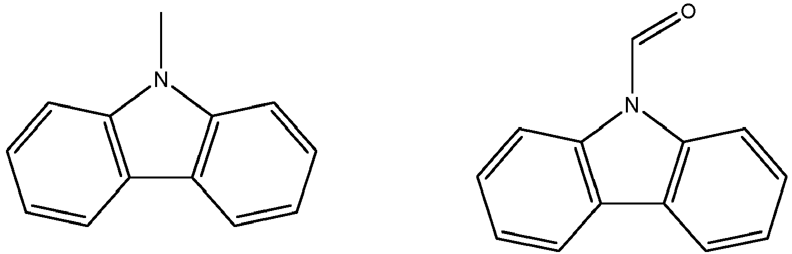

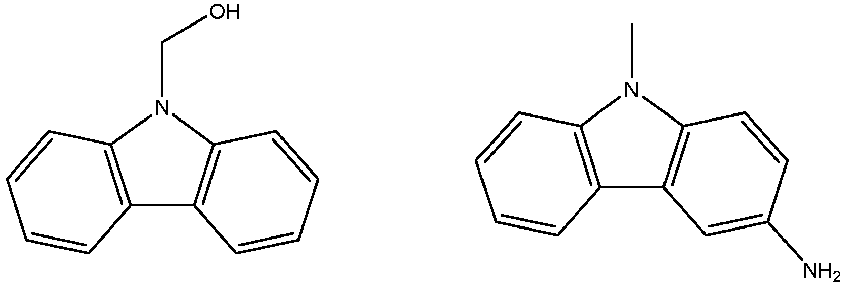

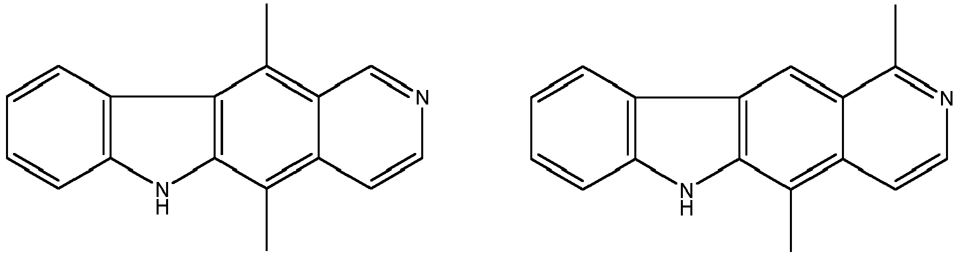

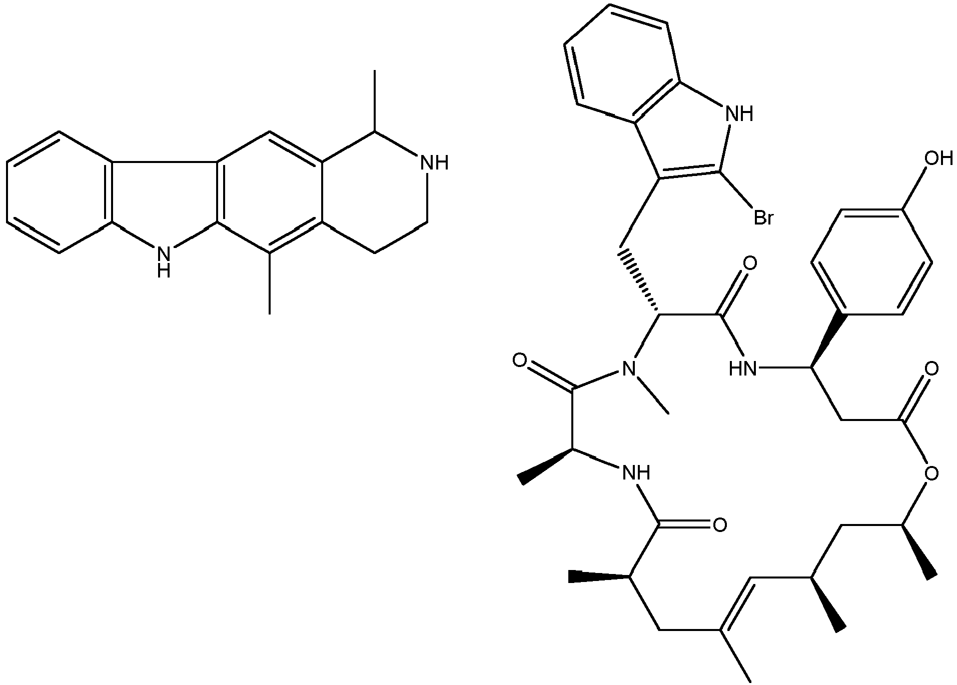

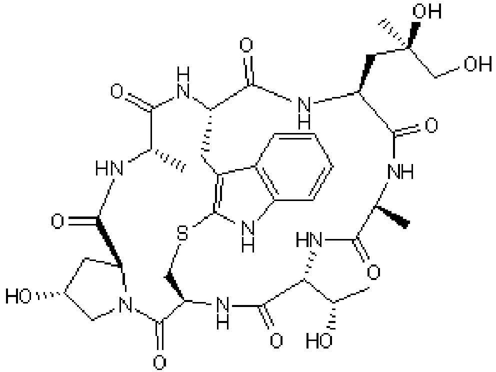

In another embodiment of the pharmaceutical combination of the present invention, the indole-containing compound is a β-carboline compound selected from the group comprising

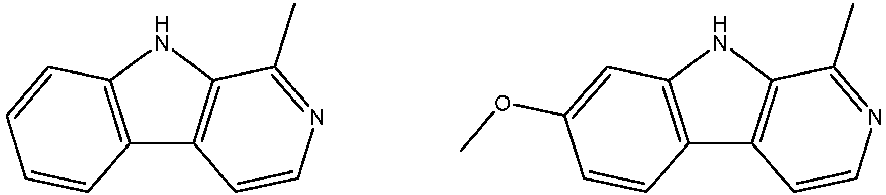

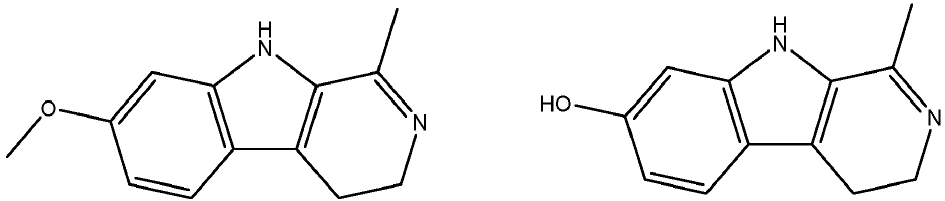

In a preferred embodiment of the pharmaceutical combination of the present invention, the indole-containing compound is harmine

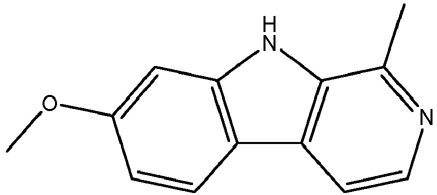

In another embodiment of the pharmaceutical combination of the present invention, the indole-containing compound is a carbazole compound selected from the group comprising

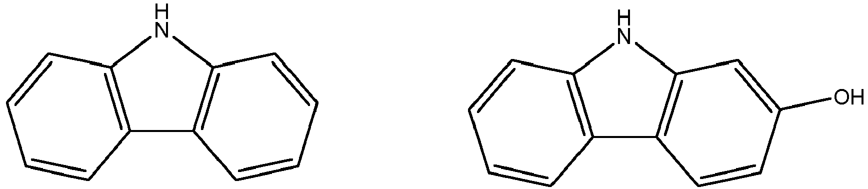

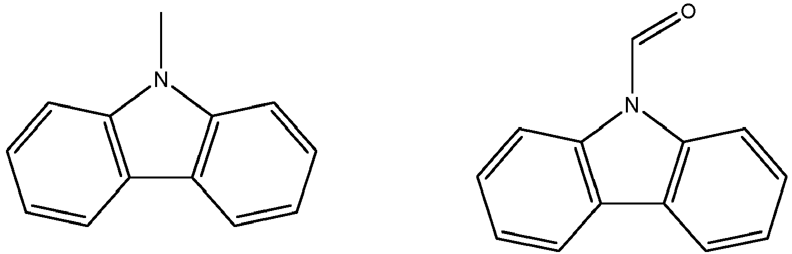

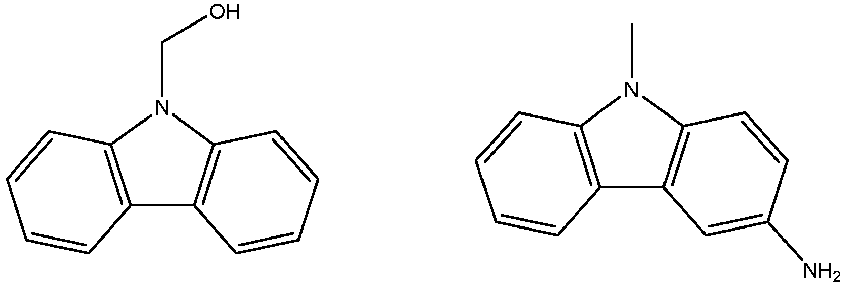

In another embodiment of the pharmaceutical combination of the present invention, the indole-containing compound is a pyridocarbazole compound selected from the group comprising

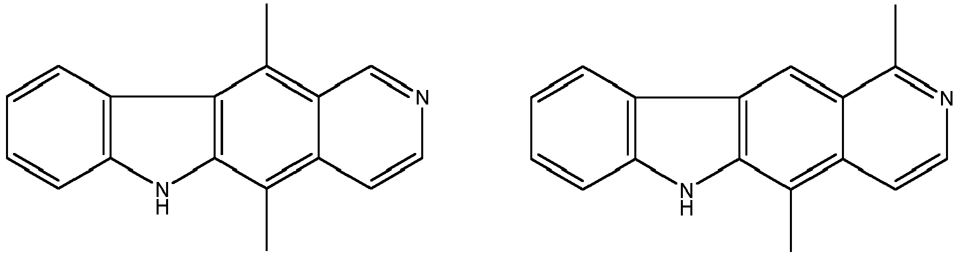

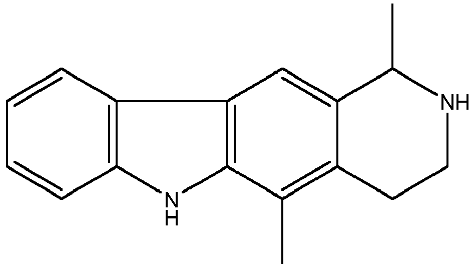

In an embodiment of the pharmaceutical combination of the present invention, the immune checkpoint inhibitor is an inhibitor of PD-1, PD-L1, PD-L2, PD-L3, PD-L4, CTLA-4, LAG3, B7-H3, B7-H4, KIR or TIM3. In another embodiment, the immune checkpoint inhibitor is selected from the group comprising nivolumab, pembrolizumab, pidilizumab, ipilimumab, dacarbazine, BMS 936559, atezolizumab, lambrolizumab, avelumab, durvalumab, or any combinations thereof.

As used herein, the term "immune checkpoint inhibitor" or "checkpoint inhibitor" refers to molecules that totally or partially reduce, inhibit, interfere with or modulate one or more checkpoint proteins. Checkpoint proteins regulate T-cell activation or function. Central to the immune checkpoint process are the cytotoxic T-lymphocyte-associated antigen 4 (CTL.A-4) and programmed death 1 (PD-1) immune checkpoint pathways. The CTL-A-4 and PD-1 pathways are thought to operate at different stages of an immune response. CTL.A-4 is considered the "leader" of the immune checkpoint inhibitors, as it stops potentially autoreactive T cells at the initial stage of naive T-cell activation, typically in lymph nodes. The PD-1 pathway regulates previously activated T cells at the later stages of an immune response, primarily in peripheral tissues.

Inhibition of the immune checkpoint pathways has led to the approval of several new drugs: ipilimumab (anti-CTI_A-4), pembrolizumab (anti-PD-1, and nivolumab (anti-PD-1). Also PD-L1 inhibitors, such as atezolizumab (MPDL3280), avelumab (MSB0010718C) and durvalumab (MEDI4736), are available. These antagonistic antibodies have been associated with objective clinical responses in cancer patients. Antibodies targeting CTL.A-4 are already marketed (e.g. ipilimumab for metastatic melanoma. Antibody therapies with anti PD-L1 (e.g. MPDL3280A), anti PD-1 (e.g. Nivolumab) are also present.

Other immune-checkpoint inhibitors include lymphocyte activation gene-3 (LAG-3) inhibitors, such as IMP321, a soluble Ig fusion protein. Other immune-checkpoint inhibitors include B7 inhibitors, such as B7-H3 and B7-H4 inhibitors. In particular, the anti-B7-H3 antibody MGA271. Also included are TIM3 (T-cell immunoglobulin domain and mucin domain 3) inhibitors.

In certain embodiments the PD-1 blockers include anti-PD-Ll antibodies. In certain other embodiments the PD-1 blockers include anti-PD-1 antibodies and similar binding proteins such as nivolumab (MDX 1106, BMS 936558, ONO 4538), a fully human IgG4 antibody that binds to and blocks the activation of PD-1 by its ligands PD-L1 and PD-L2; lambrolizumab (MK-3475 or SCH 900475), a humanized monoclonal IgG4 antibody against PD-1 ; CT-011 a humanized antibody that binds PD-1 ; AMP-224 is a fusion protein of B7-DC; an antibody Fc portion; BMS-936559 (MDX- 1105-01) for PD-L1 (B7-H1) blockade. Further examples of PD-L1 inhibitors that can be used in certain embodiments are atezolizumab (MPDL3280), Avelumab (MSB0010718C) and durvalumab.

Preferably, anti-PD-1 antibodies pembrolizumab (Keytruda), and nivolumab (Opdivo) are used in the invention. Also PD-L1 inhibitors, such as durvalumab can be used in combination with anti-PD-1-antibodies. The preferred checkpoint inhibitors of the present invention are thus those for PD-1 and PD-L1.

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. The term "PD-1" as used herein includes human PD-1 (hPD-1), variants, isoforms, and species homologs of hPD-1, and analogues having at least one common epitope with hPD-1.

CTLA-4 (cytotoxic T-lymphocyte-associated protein 4) is a protein receptor that, functioning as an immune checkpoint, downregulates the immune system. CTLA4 is found on the surface of T cells, is also a member of the immunoglobulin (Ig) superfamily; CTLA-4 comprises a single extracellular Ig domain. CTLA-4 transcripts have been found in T cell populations having cytotoxic activity, suggesting that CTLA-4 might function in the cytolytic response.

In a further embodiment, the pharmaceutical combination of the present invention further comprises one or more pharmaceutically acceptable excipients and/or carriers.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, for example, in Gilman et al. (Eds.) (1990); Goodman and Gilman's: The Pharmacological Basis of Therapeutics, 8th Ed., Pergamon Press, which is incorporated herein by reference in its entirety.

Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials may be included, which do not substantially interfere with the inhibitory activity of the compound or composition. The amount of carrier employed in conjunction with the compound or composition is sufficient to provide a practical quantity of material for administration per unit dose of the compound. Techniques and compositions for making dosage forms useful in the methods described herein are described in the following references, all incorporated by reference herein: Modern Pharmaceutics, 4th Ed., Chapters 9 and 10 (Banker & Rhodes, editors, 2002); Lieberman et ah, Pharmaceutical Dosage Forms: Tablets (1989); and Ansel, Introduction to Pharmaceutical Dosage Forms 8th Edition (2004).

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The pharmaceutical combinations of the present invention are preferably provided in unit dosage form. As used herein, a "unit dosage form" is a composition containing an amount of an indole-containing compound able to induce actin network remodelling in cancer cells into actin organized network or one or more cancer immunotherapeutic agent that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, although a single administration is not specifically excluded. The person skilled in the art will recognize that the formulation does not specifically contemplate the entire course of therapy and such decisions are left for those skilled in the art of treatment rather than formulation.

The pharmaceutical combination of the present invention may be in any of a variety of suitable forms for a variety of routes for administration, for example, for oral, sublingual, buccal, nasal, rectal, topical (including transdermal and intradermal), ocular, intracerebral, intracranial, intrathecal, intra-arterial, intravenous, intramuscular, or other parental routes of administration. Oral and parenteral administrations are customary in treating the indications that are the subject of the preferred embodiments. The person skilled in the art will appreciate that oral and nasal compositions include compositions that are administered by inhalation, and made using available methodologies.

Various oral dosage forms can be used, including such solid forms as tablets, capsules (for example solid gel capsules and liquid gel capsules), granules and bulk powders. Tablets can be compressed, tablet triturates, enteric-coated, sugar-coated, film-coated, or multiple-compressed, containing suitable binders, lubricants, diluents, disintegrating agents, colouring agents, flavouring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, colouring agents and flavouring agents.

The pharmaceutically-acceptable carriers suitable for the preparation of unit dosage forms for peroral administration is well-known in the art. Tablets typically comprise conventional pharmaceutically-compatible adjuvants as inert diluents, such as calcium carbonate, sodium carbonate, mannitol, lactose and cellulose; binders such as starch, gelatine and sucrose; disintegrants such as starch, alginic acid and croscarmelose; lubricants such as magnesium stearate, stearic acid and talc. Glidants such as silicon dioxide can be used to improve flow characteristics of the powder mixture. Colouring agents, such as the FD&C dyes, can be added for appearance. Sweeteners and flavouring agents, such as aspartame, saccharin, menthol, peppermint, and fruit flavours, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL C-591, tragacanth and sodium alginate; typical wetting agents include lecithin and polysorbate 80; and typical preservatives include methyl paraben and sodium benzoate. Peroral liquid compositions may also contain one or more components such as sweeteners, flavouring agents and colorants disclosed above.

Such compositions may also be coated by conventional methods, typically with pH or time-dependent coatings, such that the subject composition is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methylcellulose phthalate, ethyl cellulose, Eudragit coatings, waxes and shellac.

Another aspect of the present invention provides a non-cytotoxic cancer immunotherapy method, comprising administering to the subject in need thereof a therapeutically effective amount of an indole-containing compound able to induce actin network remodelling in cancer cells into actin organized network, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

The present invention also provides an indole-containing compound able to induce actin network remodelling in cancer cells into actin organized network for use in a non-cytotoxic cancer immunotherapy method, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

In an embodiment of the present invention, the non-cytotoxic cancer immunotherapy method further comprising administering one or more cancer immunotherapeutic agent before administering of the indole-containing compound and/or during administering the indole-containing compound, and/or after administering the indole-containing compound, wherein the cancer immunotherapeutic agent is selected from the group comprising immune checkpoint inhibitor, TCR-T cells, CAR-T cells or combinations thereof; preferably the cancer immunotherapeutic agent is an immune checkpoint inhibitor.

The one or more cancer immunotherapeutic agent can be engineered T-cells (TCR-T cells) expressing specific T cell receptor (TCR) and/or chimeric antigen receptor T cells (CAR-T cells) to be used as adoptive cell transfer (ACT) strategy. The one or more cancer immunotherapeutic agent can be also one or more immune checkpoint inhibitor.

In the context of the present invention, the non-cytotoxic cancer immunotherapy relates to immune modulating agents that are designed to overcome the cancer tissue-associated immune suppression. These agents enable the innate abilities of the human immune system to combat the cancer. Such therapies include cell-based therapies (e.g., ex vivo expanded, patient derived cancer neoantigen-specific T lymphocytes, T lymphocytes with genetically engineered T cell receptor (TCR), and chimeric antigen receptor (CAR)-T cell technology), oncolytic viruses (OV), monoclonal and genetically engineered antibodies (e.g., bi-specific T cell engager (BiTE) technology and immune checkpoint inhibitors (ICI)). These therapies are intended to fight cancer more effectively at both the primary cancer and at distant metastases, resulting in whole-body treatments with longer-term efficacy than cytotoxic therapies, such as chemotherapy or radiation.

Another aspect of the present invention provides a method of enhancing immune checkpoint inhibitor therapy, comprising administering to a subject in need thereof a therapeutically effective amount of an indole-containing compound able to induce actin network remodelling in cancer cells into actin organized network, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts. In a specific embodiment, the method of enhancing immune checkpoint inhibitor therapy of the invention is for non-responder patients after immune checkpoint inhibitors treatment or for patients in relapse after immune checkpoint inhibitors treatment.

The present invention also provides an indole-containing compound able to induce actin network remodelling in cancer cells into actin organized network for use in a method of enhancing immune checkpoint inhibitor therapy, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts. In a specific embodiment, the method of enhancing immune checkpoint inhibitor therapy of the invention is for non-responder patients after immune checkpoint inhibitors treatment or for patients in relapse after immune checkpoint inhibitors treatment.

In an embodiment of the method of enhancing immune checkpoint inhibitor therapy, the indole-containing compound is administered before administering the immune checkpoint inhibitor, and/or during administering the immune checkpoint inhibitor, and/or after administering the immune checkpoint inhibitor.

Another aspect of the present invention provides a method of tumour reversion, comprising administering to the subject in need thereof a therapeutically effective amount of an indole-containing compound able to induce actin network remodelling in cancer cells into actin organized network in accordance with the present invention.

The present invention also provides an indole-containing compound able to induce actin network remodelling in cancer cells into actin organized network in accordance with the present invention for use in a method of tumour reversion.

Tumour reversion results in the loss of selective advantages characterizing the tumour phenotype. Tumour reversion is accompanied by a loss of the ability of cell to grow in a semi-solid medium. Tumour reversion results as well in the relocalization of the cadherin/b-catenin complex at the cell membrane. The consequence is a rescue of cell adhesion, a decrease of cell proliferation rate and a decrease of cell motility. Tumour reversion also results in the increase in the MHC-1 expression at the tumour cell membrane and stabilizes the orientation of the MHC-1/neoantigen peptides complex. MHC-1 functionality has been found to be a prerequisite for the checkpoint inhibitors efficacy.

Another aspect of the present invention provides a method of activating cancer immune response, comprising administering the pharmaceutical combination of the invention to a subject in need thereof.

The present invention also provides the pharmaceutical combination of the invention for use in a method of activating cancer immune response.

Another aspect of the present invention provides a method of activating cancer immune response, comprising administering to the subject in need thereof a therapeutically effective amount of an indole-containing compound able to induce actin network remodelling in cancer cells into actin organized network, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

The present invention also provides an indole-containing compound able to induce actin network remodelling in cancer cells into actin organized network for use in a method of activating cancer immune response, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

Another aspect of the present invention provides a non-cytotoxic method of treating or lessening the symptoms of human cancer, or preventing human cancer progression, comprising administering the pharmaceutical combination of the invention to a subject in need thereof.

The present invention also provides the pharmaceutical combination of the invention for use in a non-cytotoxic method of treating or lessening the symptoms of human cancer, or preventing human cancer progression.

Another aspect of the present invention provides a non-cytotoxic method of treating or lessening the symptoms of human cancer, or preventing human cancer progression, comprising administering to the subject in need thereof a therapeutically effective amount of an indole-containing compound able to induce actin network remodelling in cancer cells into actin organized network, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

The present invention also provides an indole-containing compound able to induce actin network remodelling in cancer cells into actin organized network for use in a non-cytotoxic method of treating or lessening the symptoms of human cancer, or preventing human cancer progression, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

Preventing (or inhibiting) progression of the cancer is particularly important for preventing the spread of the cancer and/or metastasis, for example the progression from Stage I to Stage II where the cancer spreads locally, or the progression from Stage III to Stage IV where the cancer metastasises to other organs.

A further aspect of the present invention provides a method of activating immune response, comprising co-administering an indole-containing compound able to induce actin network remodelling in cancer cells into actin organized network in accordance with the present invention and one or more cancer immunotherapeutic agent to a subject in need thereof.

The present invention also provides an indole-containing compound able to induce actin network remodelling in cancer cells into actin organized network in accordance with the present invention for use in a method of activating immune response, wherein the indole-containing compound is co-administered with one or more cancer immunotherapeutic agent.

"Immune response" in a host refers to the development of a humoral immune response, a cellular immune response, or a humoral and a cellular immune response to an antigen. Immune responses can usually be determined using standard immunoassays and neutralization assays, which are known in the art. The term "activating immune response" refers to enhancing the level of T-cell-mediated and/or B cell-mediated immune response. In one embodiment, the level of enhancement is at least 20 50%, alternatively at least 60%, at least 70%, at least 80%, at least 90%, at least 100%, at least 120%, at least 150%, or at least 200%. In the preferred embodiment, activating immune response is accomplished via activating T-cells. The term "activating T-cells" refers to phenomenon that T cells are activated and engaged in signalling pathways that promote immune responses.

A further aspect of the present invention provides a non-cytotoxic method of treating or lessening the symptoms of human cancer, or preventing human cancer progression, comprising co-administering an indole-containing compound able to induce actin network remodelling in cancer cells in cancer cells into actin organized network in accordance with the present invention and one or more cancer immunotherapeutic agent to a subject in need thereof.

In an embodiment, the present invention provides an indole-containing compound able to induce actin network remodelling in cancer cells into actin organized network in accordance with the present invention for use in the treatment or lessening the symptoms of human cancer, or the prevention of human cancer progression, wherein the indole-containing compound is co-administered in combination with one or more cancer immunotherapeutic agent to a patient.

In another embodiment, the present invention provides the pharmaceutical combination of the present invention for use in the treatment or lessening the symptoms of human cancer, or the prevention of human cancer progression.

In the context of the present invention, the cancer candidates are those displaying major cytoskeleton disorganization. This feature corresponds to a marked undifferentiated phenotype as seen in cancers of mesenchymal origin such as leukemia and sarcoma as well as melanoma expressing high level of mesenchymal markers. In an embodiment of the methods of the present invention, the human cancer comprises cancer cells expressing a binding ligand of PD-1 or a binding ligand of CTLA-4. In a preferred embodiment, the binding ligand of PD-1 is PD-L1 or PD- L2. In another embodiment of the methods of the present invention, the human cancer is selected from the group comprising head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloma, lymphoma, or leukemia. In a further embodiment of the methods of the present invention, human cancer comprises cancer cells lacking MHC-1 selected from the group comprising oral squamous cell carcinomas, Merkel cell carcinoma, or cancer cells displaying low MHC-1 expression selected from the group comprising colon adenocarcinoma, breast cancer, bladder cancer, lung cancer, melanoma.

Some embodiments of the present invention provide a non-cytotoxic method of treating human cancer comprising administering the pharmaceutical combination of the present invention to a subject in need thereof.

Some embodiments of the present invention provide a method of providing co-stimulation of T-cell activation against cancer by co-administering an indole-containing compound able to induce actin network remodelling in cancer cells into actin organized network in accordance with the present invention and one or more cancer immunotherapeutic agent to a subject in need thereof. Some other embodiments of the present invention provide a method of providing co-stimulation of natural killer cells against cancer by co-administering an indole-containing compound able to induce actin network remodelling in cancer cells into actin organized network in accordance with the present invention and one or more cancer immunotherapeutic agent to a subject in need thereof.

In some embodiments, the non-cytotoxic method of treating human cancer of the present invention further includes identifying cancer cells expressing a binding ligand of PD-1. In some embodiments, the non-cytotoxic method of treating human cancer of the present invention further includes identifying cancer cells expressing PD-L1. In some embodiments, the non-cytotoxic method of treating human cancer of the present invention further includes identifying cancer cells expressing PD-L2. In some embodiments, the non-cytotoxic method of treating human cancer of the present invention further includes identifying cancer cells expressing PD-L3 or PD-L4.

In some embodiments, identifying cancer cells expressing a binding ligand of PD-1 includes using an assay to detect the presence of the binding ligand. Examples of applicable assay include but are not limited to PD-L1 IHC 22C3 pharmDx kit and PD-L1 IHC 28-8 pharmDx available from Dako.

In some embodiments, the cancer comprises cancer cells expressing a binding ligand of CTLA-4. In some embodiments, the binding ligand of CTLA-4 is B7.1 or B7.2.

In some embodiments, the non-cytotoxic method of treating human cancer of the present invention further includes identifying cancer cells expressing a binding ligand of CTLA-4. In some embodiments, the non-cytotoxic method of treating human cancer of the present invention further includes identifying cancer cells expressing B7.1 or B7.2.

In some embodiments, the present invention provides a use of an indole-containing compound able to induce actin network remodelling in cancer cells into actin organized network in accordance with the present invention in the treatment or lessening the symptoms of human cancer, or the prevention of human cancer progression.

In some embodiments, the present invention provides a use of an indole-containing compound able to induce actin network remodelling in cancer cells into actin organized network in accordance with the present invention in the treatment or lessening the symptoms of human cancer, or the prevention of human cancer progression, wherein the indole-containing compound is administered to a subject in need thereof in combination with one or more cancer immunotherapeutic agent.

As described above, some embodiments include co-administering an indole-containing compound able to induce actin network remodelling in cancer cells into actin organized network in accordance with the present invention and one or more cancer immunotherapeutic agent. By "co-administration" or "co-administering", it is meant that the indole-containing compound and the one or more cancer immunotherapeutic agent are administered in such a manner that administration of the indole-containing compound has an effect on the efficacy and/or safety of the cancer immunotherapeutic agent, regardless of when or how they are actually administered. Thus in one embodiment, the indole-containing compound and the one or more cancer immunotherapeutic agent are administered simultaneously. In one such embodiment, administration in combination is accomplished by combining the indole-containing compound and the one or more cancer immunotherapeutic agent in a single dosage form. In another embodiment, the indole-containing compound and the one or more cancer immunotherapeutic agent are administered sequentially. In one embodiment the indole-containing compound and the one or more cancer immunotherapeutic agent are administered through the same route, such as orally or intravenously. In another embodiment, the indole-containing compound and the one or more cancer immunotherapeutic agent are administered through different routes, such as one being administered orally and another being administered i.v. In some embodiments, the time period between administration of the indole-containing compound and administration of the co-administered one or more cancer immunotherapeutic agent can be about 1 hour, 2 hours, 3 hours, 5 hours, 8 hours, 10 hours, 12 hours, 15 hours, 1 8 hours, 20 hours, 24 hours, 36 hours, 48 hours, 3 days, 4 days, 5 days, 6 days, 7 days, 10 days, 14 days, 21 days, 28 days, or 30 days.

In some embodiments, the treatment cycle can include co-administering an indole-containing compound able to induce actin network remodelling in cancer cells into actin organized network in accordance with the present invention and one or more cancer immunotherapeutic agent in combination with administering the indole-containing compound alone or administering the one or more cancer immunotherapeutic agent alone. In some embodiments, an indole-containing compound able to induce actin network remodelling in cancer cells into actin organized network in accordance with the present invention and one or more cancer immunotherapeutic agent are co-administered on day 1 , followed by administration of the indole-containing compound alone after 1 day, 2 days, 3 days, 4 days, 5 days, 6 days, 7 days, 2 weeks, or 3 weeks, and then followed by co-administration of the indole-containing compound and one or more cancer immunotherapeutic agent after 1 day, 2 days, 3 days, 4 days, 5 days, 6 days, 7 days, 2 weeks, or 3 weeks. In some embodiments, an indole-containing compound able to induce actin network remodelling in cancer cells into actin organized network in accordance with the present invention and one or more cancer immunotherapeutic agent are administered simultaneously on day 1, followed by administration of the indole-containing compound or one or more cancer immunotherapeutic agent alone on a day selected between day 2 and day 31, and then followed by co-administration of the indole-containing compound and one or more cancer immunotherapeutic agent on a day selected between day 3 and day 31. In some embodiments, an indole-containing compound able to induce actin network remodelling in cancer cells into actin organized network in accordance with the present invention and one or more cancer immunotherapeutic agent are co-administered on day 1, followed by administration of the indole-containing compound alone on day 8, and then followed by co-administration of the indole-containing compound and one or more cancer immunotherapeutic agent on day 15. In some embodiments, the treatment cycle can be repeated two or more times.

In other embodiments, an indole-containing compound able to induce actin network remodelling in cancer cells into actin organized network in accordance with the present invention is administered before administering of the one or more cancer immunotherapeutic agent and/or an indole-containing compound able to induce actin network remodelling in cancer cells into actin organized network in accordance with the present invention is administered during administering of the one or more cancer immunotherapeutic agent and/or after administering the one or more cancer immunotherapeutic agent.

In an embodiment of the methods of the present invention, the indole-containing compound is selected from the group comprising

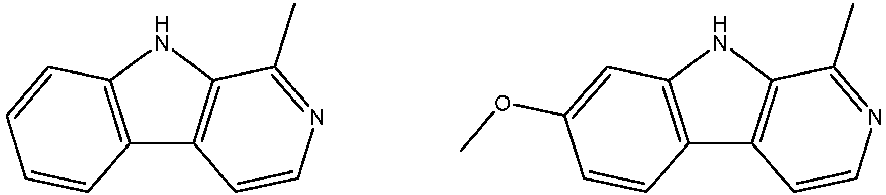

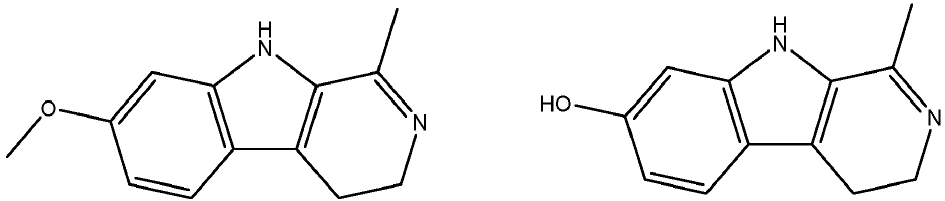

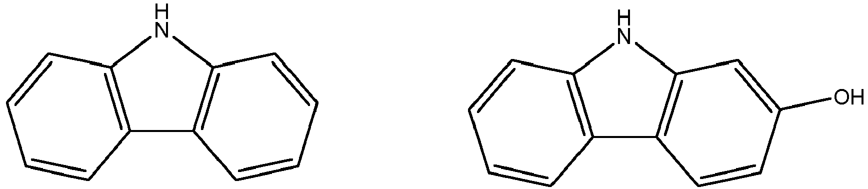

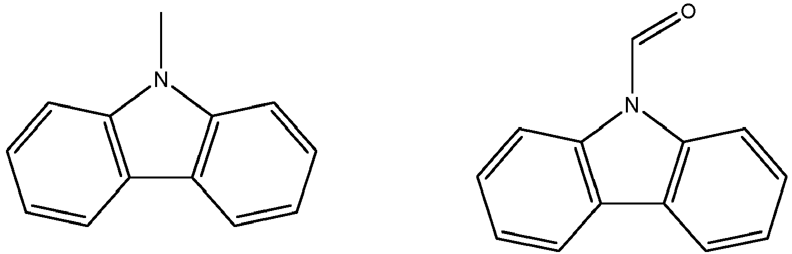

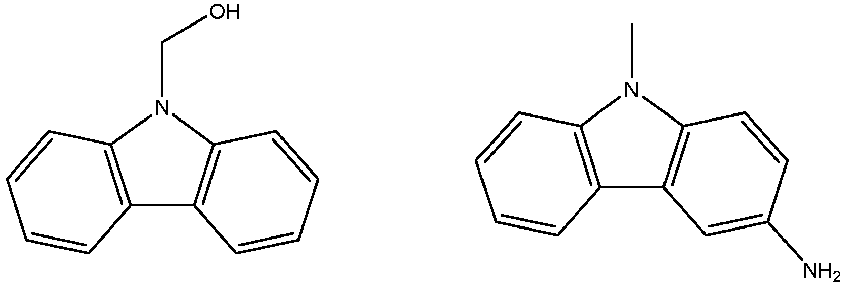

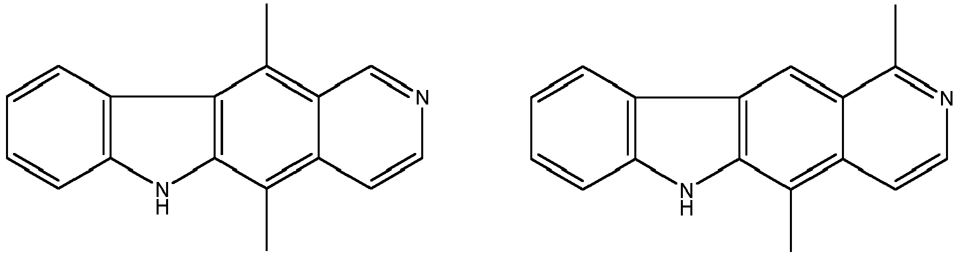

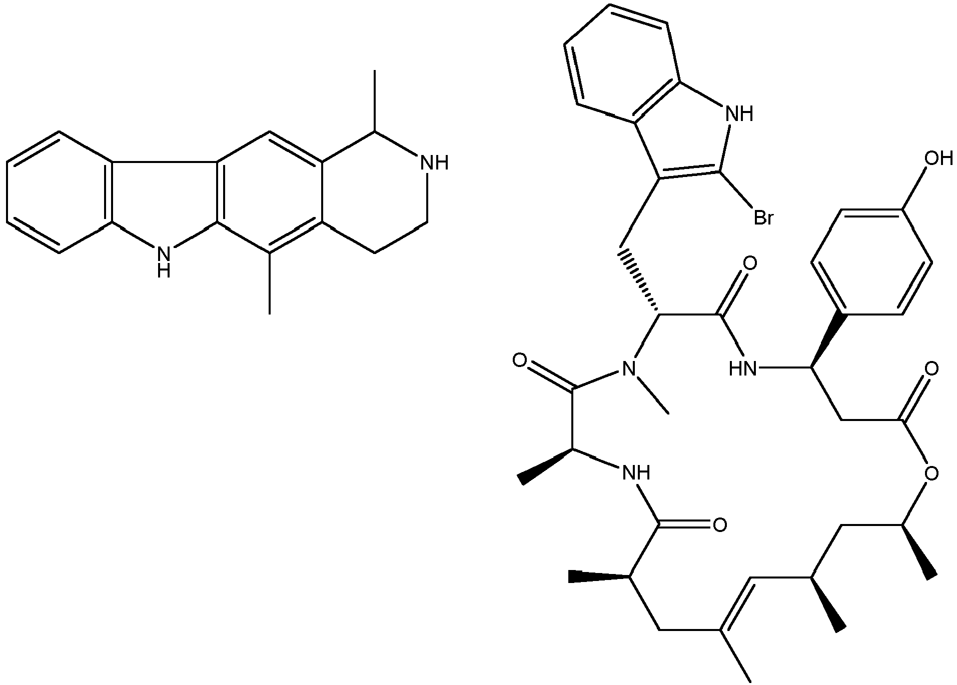

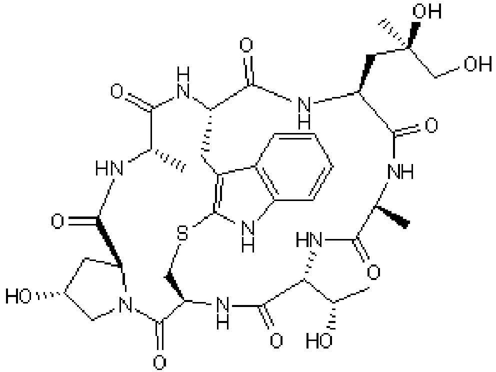

In another embodiment of the methods of the present invention, the indole-containing compound is a β-carboline compound selected from the group comprising

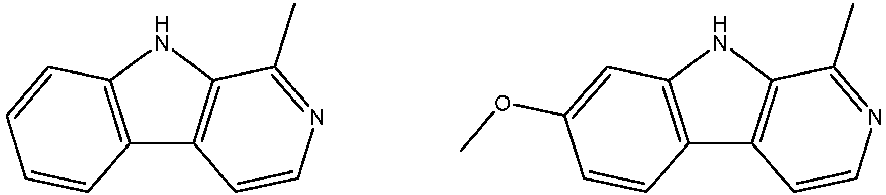

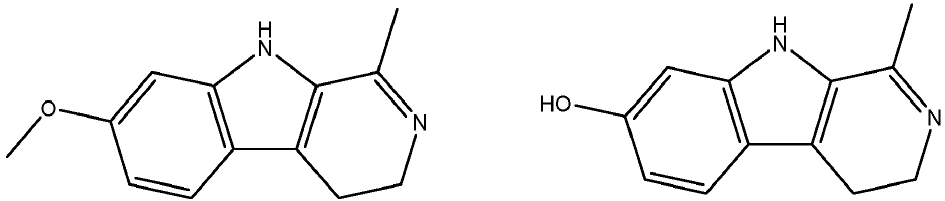

In a preferred embodiment of the method of the present invention, the indole-containing compound is harmine

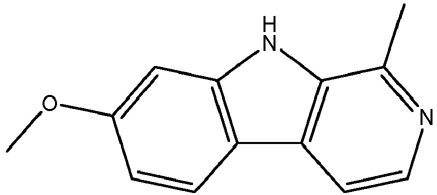

In another embodiment of the method of the present invention, the indole-containing compound is a carbazole compound selected from the group comprising

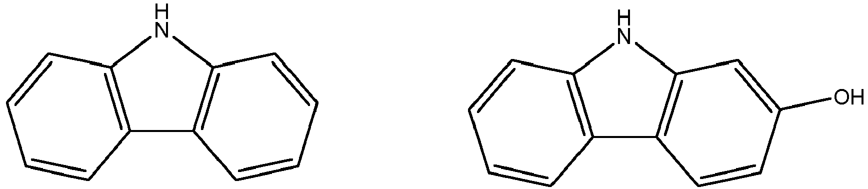

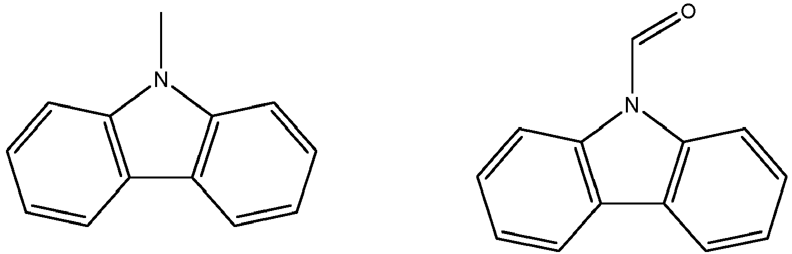

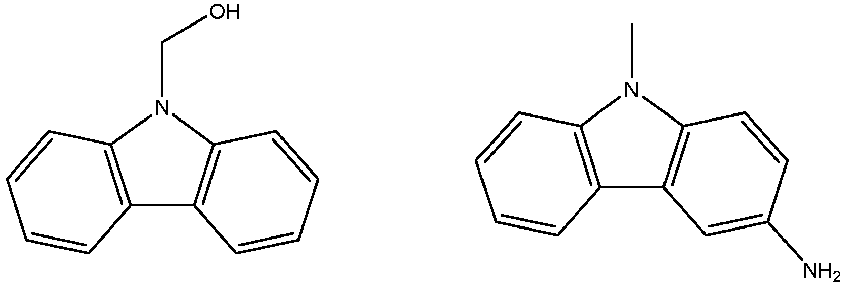

In another embodiment of the method of the present invention, the indole-containing compound is a pyridocarbazole compound selected from the group comprising

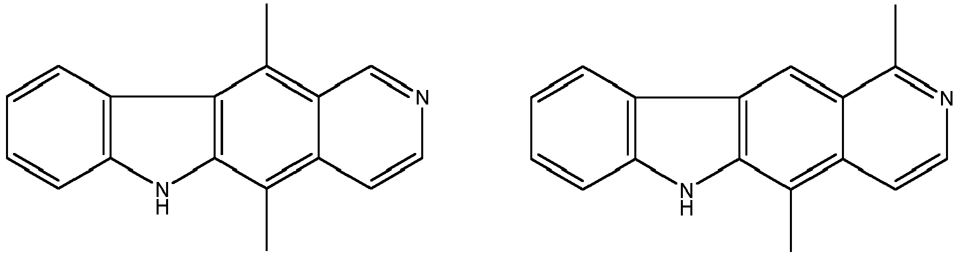

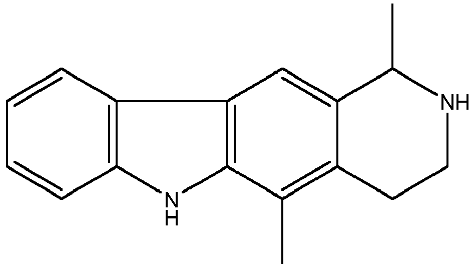

The advantages of the non-cytotoxic methods of the invention, such as the method of activating cancer immune response, the method of treating or lessening the symptoms of human cancer, or preventing human cancer progression, the cancer immunotherapy method and the method of enhancing immune checkpoint inhibitor therapy, is that such non-cytotoxic methods can be provided to patients with advanced cancer in failure of any other treatment and to fragile patients who do not tolerate cytotoxic anticancer therapies, such as chemotherapy or therapies using cytotoxic drugs.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended claims, and all changes which come within the meaning and range of equivalency are intended to be embraced therein.

The foregoing description will be more fully understood with reference to the following Examples. Such Examples, are, however, exemplary of methods of practising the present invention and are not intended to limit the application and the scope of the invention.

### EXAMPLES

To study tumour reversal, the Applicant has discovered that the treatment of malignant fibroblasts by a methanolic extract ACB-1E from *Peganum Harmala* resulted in actin cytoskeleton remodelling, recovery of cell-cell adhesion and ultimately in the loss of malignant properties (figure 1).

It has been further observed that the recovery of cell-cell adhesion as induced by the treatment of cells with the methanolic extract of ACB-1E was accompanied by the relocalization of β-catenin at the juxtamenbrane region (figure 2).

In malignant isolated cells, β-catenin accumulates in the cytosol and can be translocated to the nucleus, activating so, genes involved in cell proliferation such as cyclin D1.

It has been found that the methanolic extract of ACB-1E was able to increase actin polymerization in cell extract of malignant fibroblasts (Figure 3). In malignant fibroblasts, the dynamics of actin polymerization is markedly lower than the one observed in parental non-malignant NIH-3T3 fibroblasts. When ACB-1E methanolic extract was added to the malignant cell extract, the dynamics of actin polymerization displays similar parameters (rate constant and plateau value) as observed in non-malignant NIH-3T3. This property may explain the reorganization of the actin cytoskeleton in malignant fibroblasts as shown in figures 1 and 2.

The identification of the active methanolic extract was the result of an extensive screening aiming at the identification of compounds of natural origin able to act as antitumor agent. The selected ACB-1E methanolic extract was from *Peganum Harmala* which contain various alkaloids mainly identified as β-carbolin compounds including Harmine, Harman, Harmalol, Harmaline.

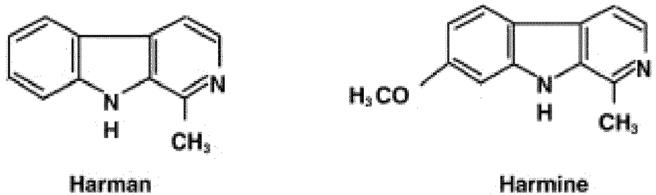

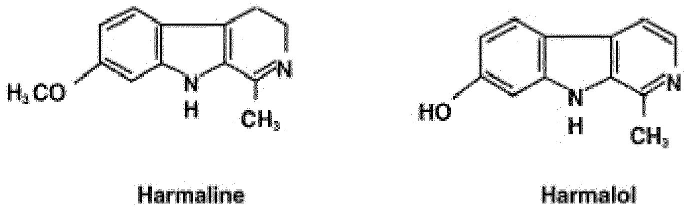

It has been further identified that the active molecule present in the ACB-1E methanolic extract was Harmine (ACB1801). Harmine (7-Methoxy-1-methyl-9H-pyrido[3,4-b]-indole) belongs to the β-carboline family of compounds and is a psychoactive and vasorelaxant drug that also has a cytopathic activity against cancer cells. Harmine was found to bind to DNA with a low affinity. When treated by non-cytotoxic concentrations of Harmine, the malignant fibroblasts recover cell-cell adhesion properties and self-organize in clusters as naturally occurring with non-malignant fibroblasts (figure 4).

Major actin cytoskeleton disorganization is observed in various mesenchymal tumours including sarcomas and leukaemia such as AML and ALL as well as in invasive melanoma which express mesenchymal markers.

Figure 5 shows that the treatment of murine B16 F10 melanoma cells by ACB1801 results in a marked remodelling of the actin cytoskeleton architecture accompanied by a dramatic change of cell morphology moving from round cells to fibroblastoid shape referred to as "flat revertant" as described by Noda et al. (1989).

The cell-cell adhesion rescue following ACB1801 treatment can be objective by the measurement of the actual cell-cell adhesion forces. Data in table 1 show that ACB1801 treatment results in the recovery of malignant cell-cell adhesion forces up to the level measured in non-malignant fibroblasts.

**Table 1. Cell disruption forces of NIH-3T3 and 3T3/EF. Forces are expressed in nanoNewtons. Each value +/- SD was the mean of 20 doublets measurement performed on three independent experiments (see materials and methods). When indicated, 3T3/EF cells were incubated in the presence of 10 µM ACB1801 for 24 hours.**

| Time | NIH-3T3 | 3T3/EWS-Fli-1 | 3T3/EWS-Fli-1 + 10 µM ACB1801 |
|---|---|---|---|
| 30 sec | 5.0+/- 0.6 | 2.8+/- 0.2 | 4.9+/-0.7 |
| 4 min | 14.9+/- 1.4 | 9.5+/-1.1 | 13.5+/-1.3 |

It has been further found that the actin cytoskeleton reorganization, beyond the recovery of cell-cell adhesion, results in the loss of malignant character assessed by the inability of the ACB1801-treated cells to grow in semi solid medium. Anchorage independent growth reflects the ability of cells to survive and to proliferate in the absence of signalling from the extracellular matrix mediated principally by integrins. This feature of malignant can be assessed through evaluation of cell clonogenicity in semisolid medium (figure 6A). Cells were seeded (500 per 35-mm dish) in culture medium supplemented with methylcellulose. Clones with a diameter larger than 120µm were counted after 4 weeks. The treatment of cells with increasing concentrations of ACB1801 resulted in a marked inhibition of proliferation with an IC₅₀ of about 4.5 µM. It should be noticed that ACB1801 cytotoxicity as measured by the MTT test yielded an IC₅₀ of 18.5 µM and that consequently the inhibition of proliferation in semi solid medium is most probably not due to a direct cytotoxic effect. This assumption is confirmed by flow cytometry as shown in figure 6B which depicts the effect of ACB1801 on the cell cycle of E/F cells. In the absence of drug, most cells (84%) were in the G1 and S phases due to the proliferative state of E/F cells. After 72h of incubation with 1µM, 5µM and 10µM ACB1801, no significant variation in the cell cycle was observed and in particular no sub-Gl peak, characteristic of apoptotic cells, was detected. The absence of a ACB1801 induced cytotoxic effect was confirmed by the absence of apoptosis, assessed by annexin V-FITC staining in cells treated for 72 hours with ACB1801 at concentrations of up to 10 µM, (data not shown). In contrast, both cell cycle alteration and apoptosis were observed in cells treated with 50 µM Harmine.

It is concluded that the cloning inhibition is not due to cytotoxic process but due to the loss of malignant character. This is confirmed by the fact that malignant fibroblasts pre-treated with 5µM Harmine for 48h lose their ability to form tumour in mice (data not shown).

In contrast to F-actin stabilizing molecules such as Phalloidine or Jasplakinolide, Harmine do not bind to F-actin. This suggests that Harmine interacts with key regulators involved in the signalling of actin dynamics. Performing a kinome scan experiment, it has been found that Harmine interacts with several kinases: HIPK1, HIPK2, DIRK1A, DIRK1B, CDK7, CDK9.

The functionality of all proteins expressed at the cell membrane and involved in the immunological synapse between tumour cells and TCL, including the TCR and MHC-1 are under the dependence of the actin organization. The appropriated protein orientation and the adhesion forces depends on the integrity of the cortical F-actin network. F-actin allows the structural organization and localization of proteins such as filamin which interacts with the intracellular domain of the various proteins involved in the immunological synapse. Figure 6 shows the structural organization of MHC-1 and the colocalization of MHC I and filamin. Figure 7A shows in turn the colocalization of MHC I and actin and figure 7B shows that the treatment of cells with interferon gamma which is known to increase the MHC I density at the cell membrane is accompanied by a significant increase of actin density.

All these observations indicate that the actin network disorganization in malignant cells participates to the impairment of MHC I functionality and that the recovery of a convenient cytoskeleton organization will improve molecular communication between reverting tumour cells and CD8 TCL.

According the hypothesis, the F-actin network rescue following the treatment of melanoma B16 by 5µM harmine results in a significant increase of HMC I level at the cell membrane (figure 9). In contrast to interferon gamma, harmine has a little effect on the MHC I genes expression level suggesting that the increase at the membrane level is likely due to the stabilisation of the complex through filamin/actin supra molecular structure.

It was further observed that harmine displays a limited effect on PD-L1 expression at the membrane of melanoma B16 cells and that in contrast with the huge effect observed following the interferon gamma treatment. This can be considered as a favourable property because PD-L1 overexpression in tumour cells is constantly associated with poorer overall survival in cancer patients.

Further experiments have been performed in syngeneic mice grafted with melanoma B16 cells. It was known that in this experimental model the anti PD1 antibody has no effect on tumour growth. This inefficacy was verified as shown in figure 11. The anti PD1 inefficacy is not due to a lack of PD-L1 expression as shown in figure 10 but probably to an abnormal expression and positioning of the MHCl complex at the tumour cell membrane. The daily oral administration of 50 mg/kg ACB-1801 (harmine) to mice bearing melanoma B16 results in a significant antitumor effect mainly coming from the impairment of the tumour phenotype referred to as tumour reversion. **The striking point is that in harmine treated mice, the anti PD1 antibody recovered a significant antitumor activity and the combination of ACB-1801 (harmine) and anti PD1 induces a strong synergistic effect.**

## Claims

1. A pharmaceutical combination of an indole-containing compound able to induce actin network remodelling in cancer cells into actin organized network and one or more cancer immunotherapeutic agent for simultaneous, separate or sequential administration,
wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts,
and wherein the cancer immunotherapeutic agent is selected from the group comprising immune checkpoint inhibitor, TCR-T cells, CAR-T cells or combinations thereof; preferably the cancer immunotherapeutic agent is an immune checkpoint inhibitor.

2. The pharmaceutical combination of claim 1, wherein the indole-containing compound is selected from the group comprising

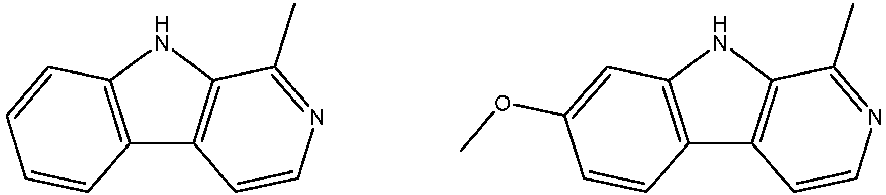

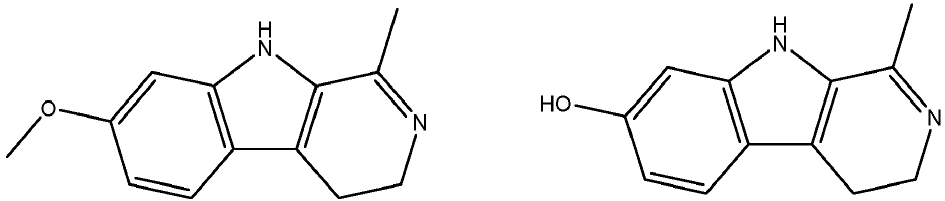

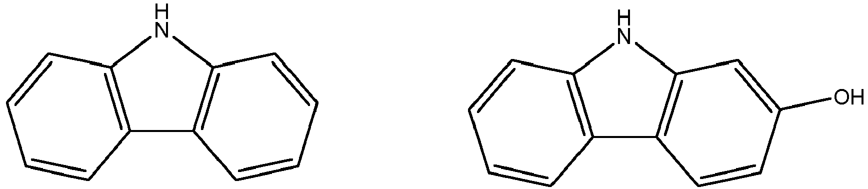

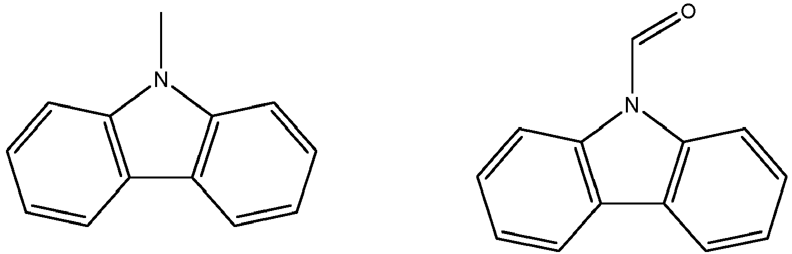

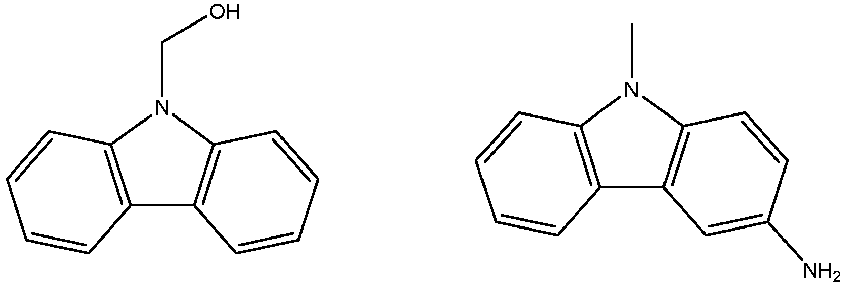

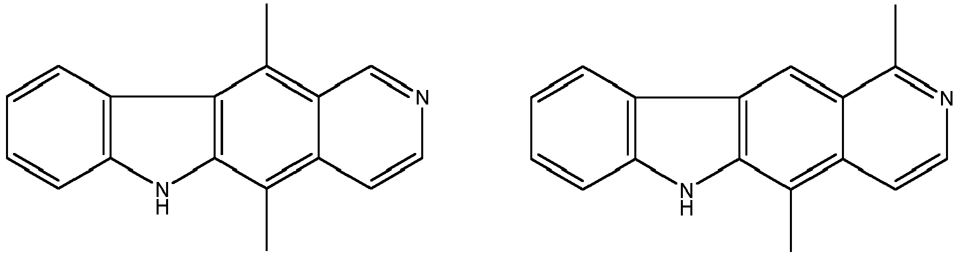

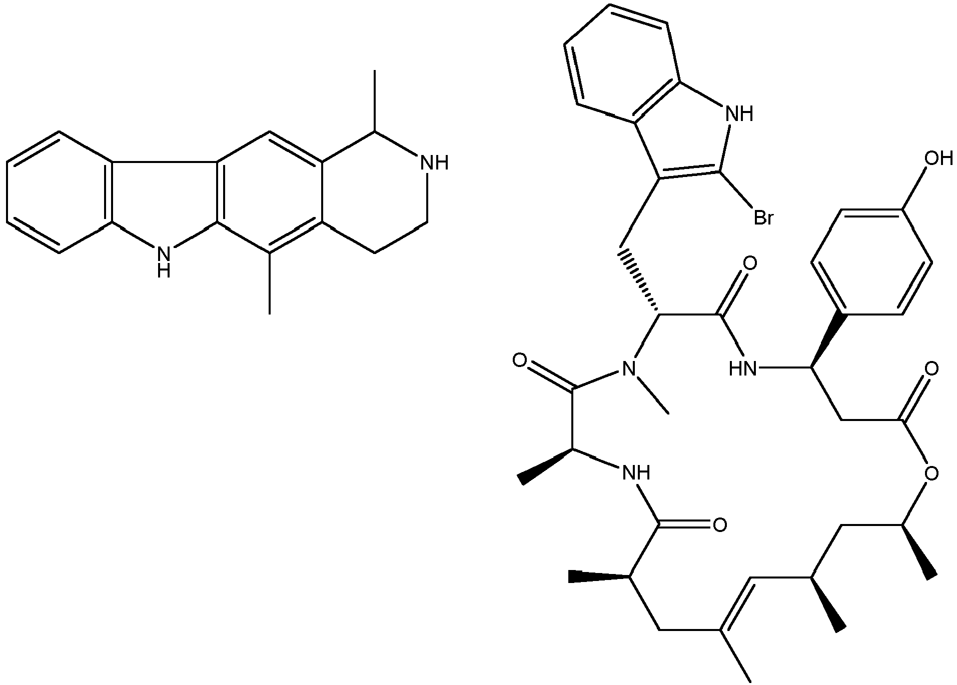

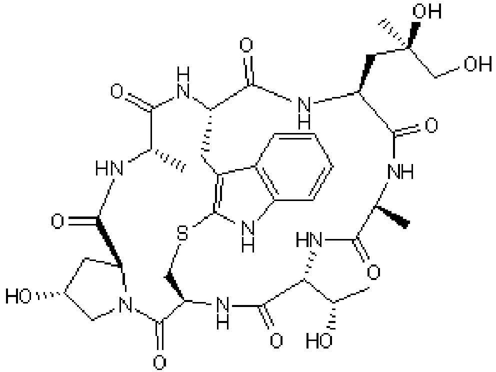

3. The pharmaceutical combination of claim 1, wherein the indole-containing compound is

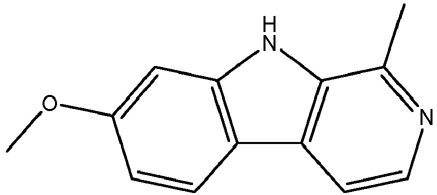

4. The pharmaceutical combination of any one of claims 1-3, wherein the immune checkpoint inhibitor is selected from the group comprising nivolumab, pembrolizumab, pidilizumab, ipilimumab, dacarbazine, BMS 936559, atezolizumab, lambrolizumab, avelumab, durvalumab, or any combinations thereof.

5. A pharmaceutical combination of any one of claims 1-4 for use in a method of activating cancer immune response.

6. A pharmaceutical combination of any one of claims 1-4 for use in non-cytotoxic method of treating or lessening the symptoms of human cancer, or preventing human cancer progression.

7. The pharmaceutical combination for use according to claim 6, wherein the human cancer comprises cancer cells lacking MHC-1 selected from the group comprising oral squamous cell carcinomas, Merkel cell carcinoma, or cancer cells displaying low MHC-1 expression selected from the group comprising colon adenocarcinoma, breast cancer, bladder cancer, lung cancer, melanoma.

8. An indole-containing compound able to induce actin network remodelling in cancer cells into actin organized network for use in a non-cytotoxic cancer immunotherapy method, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

9. The indole-containing compound able to induce actin network remodelling in cancer cells into actin organized network for use according to claim 8, wherein the indole-containing compound is co-administered with one or more cancer immunotherapeutic agent before administering the indole-containing compound and/or during administering the indole-containing compound, and/or after administering the indole-containing compound, wherein the cancer immunotherapeutic agent is selected from the group comprising immune checkpoint inhibitor, TCR-T cells, CAR-T cells or combinations thereof; preferably the cancer immunotherapeutic agent is an immune checkpoint inhibitor.

10. The indole-containing compound able to induce actin network remodelling in cancer cells into actin organized network for use according to claim 9, wherein the immune checkpoint inhibitor is selected from the group comprising nivolumab, pembrolizumab, pidilizumab, ipilimumab, dacarbazine, BMS 936559, atezolizumab, lambrolizumab, avelumab, durvalumab, or any combinations thereof.

11. An indole-containing compound able to induce actin network remodelling in cancer cells into actin organized network for use in a method of activating cancer immune response, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

12. An indole-containing compound able to induce actin network remodelling in cancer cells into actin organized network for use in a non-cytotoxic method of treating or lessening the symptoms of human cancer, or preventing human cancer progression, wherein the indole-containing compound fulfils the following criteria: when malignant fibroblasts (E/F) extract is grown in presence of the indole-containing compound, the dynamics of actin polymerization displays similar parameters (rate constant and plateau value) as observed in non-malignant fibroblasts, and the malignant fibroblasts (E/F) recover cell-cell adhesion properties and self-organize in clusters as occurring with non-malignant fibroblasts.

13. The indole-containing compound able to induce actin network remodelling in cancer cells into actin organized network for use according to claim 12, wherein the human cancer comprises cancer cells lacking MHC-1 selected from the group comprising oral squamous cell carcinomas, Merkel cell carcinoma, or cancer cells displaying low MHC-1 expression selected from the group comprising colon adenocarcinoma, breast cancer, bladder cancer, lung cancer, melanoma.

14. The indole-containing compound able to induce actin network remodelling in cancer cells into actin organized network for use according to anyone of claims 8-13, wherein the indole-containing compound is selected from the group comprising

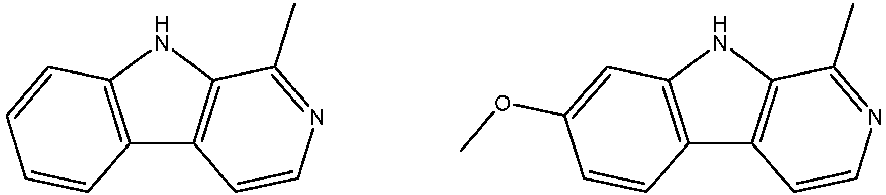

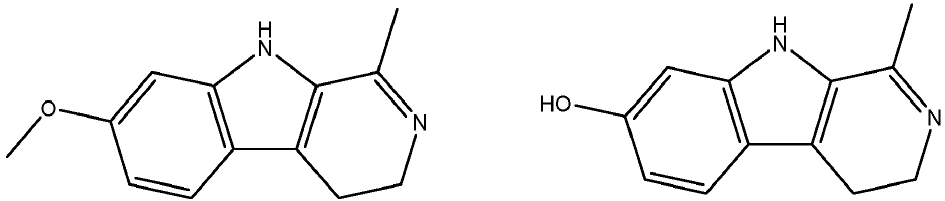

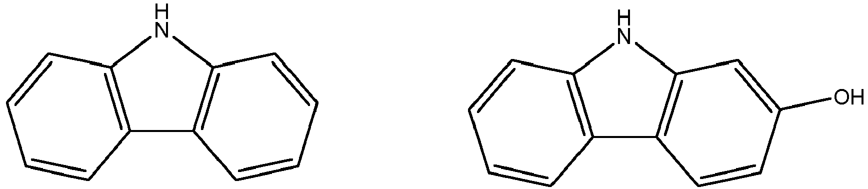

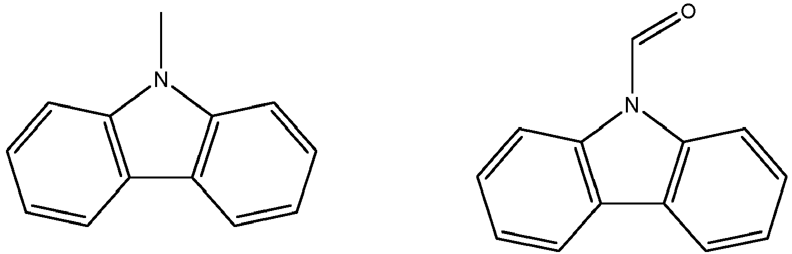

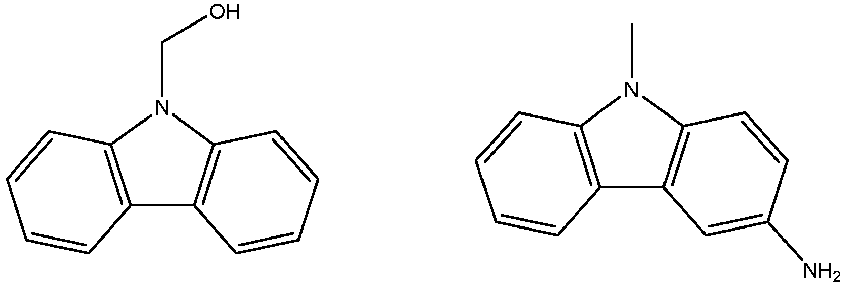

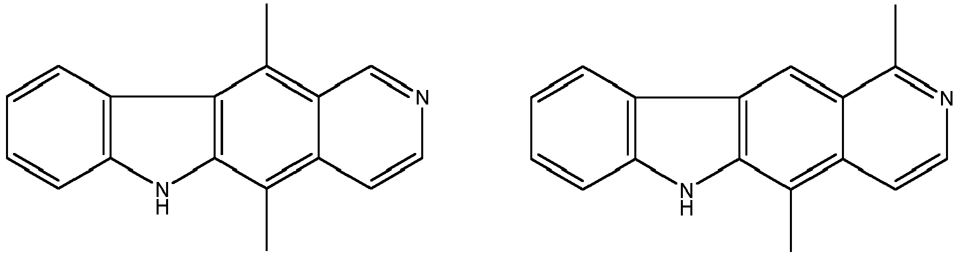

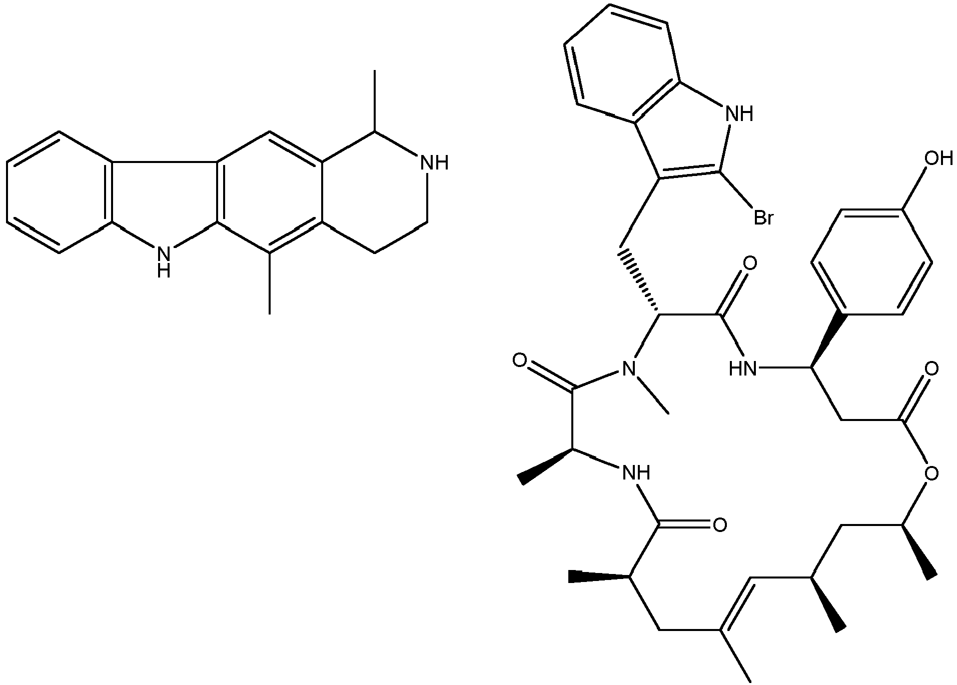

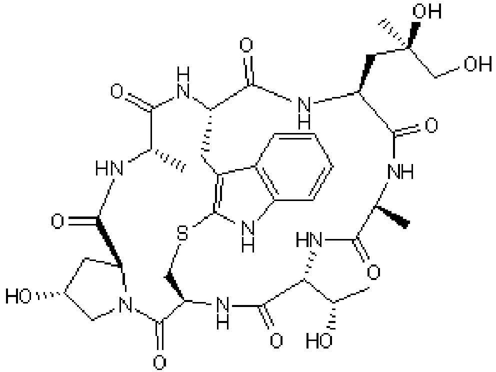

15. The indole-containing compound able to induce actin network remodelling in cancer cells into actin organized network for use according to anyone of claims 8-13, wherein the indole-containing compound is
